# EUROPEAN PATENT APPLICATION

(11) **EP 3 263 095 A1**
(43) Date of publication of application: **03.01.2018**
(21) Application number: 17020256.8
(22) Date of filing: 19.06.2017
(51) Int. Cl.: A61K 9/14, A61K 31/502

(54) **A PHARMACEUTICAL FORMULATION OF OLAPARIB**

(30) Priority: 29.06.2016 CZ 20160391
(71) Applicant: Zentiva K.S., 102 37 Praha 10 (CZ)
(72) Inventor: Ridvan, Ludek, 102 00 Praha 10 - Hostivar (CZ); Pecek, Daniel, 628 00 Brno (CZ); Tieger, Eszter, 9963 Magyarlak (HU); Zvatora, Pavel, 789 03 Plumlov (CZ); Tozickova, Hana, 155 00 Praha 5 (CZ); Svobodova, Jaroslava, 250 84 Krenice (CZ); Dammer, Ondrej, 253 01 Hostivice (CZ); Krejcik, Lukas, 190 17 Praha 9 (CZ); Hrabalová, Lucie, 338 28 Radnice (CZ); Boleslavska, Tereza, 154 00 Praha 5 (CZ)
(74) Representative: Jirotkova, Ivana

(57) **Abstract**

The present invention relates to novel pharmaceutical preparations containing olaparib of formula **I**, systematically 4-[(3-[(4-cyclopropylcarbonyl)piperazin-4-yl]carbonyl)-4-fluorophenyl]methyl(2H)-phtalazin-1-one, with enhanced bioavailability, stability, or possibly reduced dosage of the active ingredient, a preparation method of these drug forms and their use for the treatment of cancer.

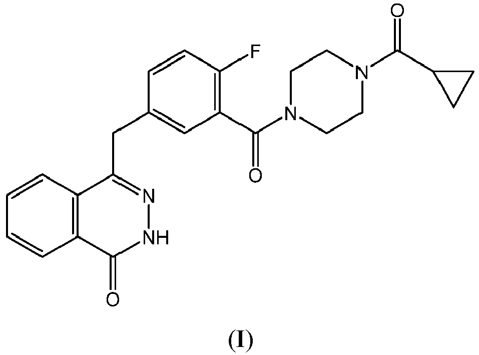

## Description

### Field of the Invention

The invention relates to novel pharmaceutical preparations containing olaparib of formula I, systematically 4-[(3-[(4-cyclopropylcarbonyl)piperazin-4-yl]carbonyl)-4-fluorophenyl]methyl(2H)-phtalazin-1-one, with enhanced bioavailability, stability, or possibly reduced dosage of the active ingredient, to a preparation method of these drug forms and their use for the treatment of cancer.

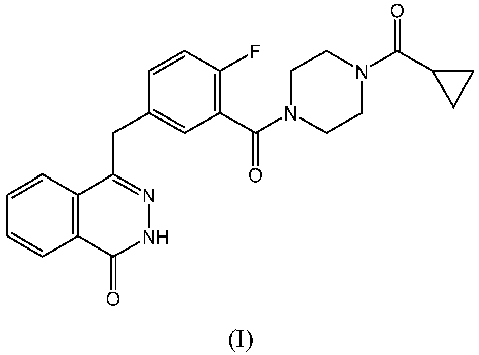

### Background Art

Olaparib, in the form of the drug preparation Lynparza®, is used for the treatment of some types of cancer (e.g. ovarian and oviduct cancer). Synthesis of olaparib was first described in patent application WO2004080976. Olaparib exhibits polymorphism, i.e. it crystallizes in various modifications that may principally differ with their physicochemical characteristics (solubility, stability etc.). Polymorphic form A, which is used in the Lynparza preparation, was characterized in patent application WO2008047082. Patent application WO2009050469 then described crystalline form L. The document of the European Medicines Agency of October 23, 2014 (EMA/CHMP/789139/2014) mentions that olaparib is a substance with low solubility and bioavailability, which means that it is classified in Group 4 of the Biopharmaceutical Classification System. The recommended daily dose of the lipid-based drug form Lynparza® 50 mg, i.e. a crystalline suspension of olaparib Form A in the fatty excipient Gelucire™, is twice 400 mg, which represents 16 capsules a day.

Patent application WO2010041051 describes various approaches to increase solubility and bioavailability of olaparib. Based on dissolutions and pharmacokinetic data, the authors infer that for the preparation of a drug form with increased bioavailability conversion of crystalline olaparib to an amorphous solid solution with polymers exhibiting low hygroscopicity and a high glass transition temperature (T_{g}) or melting point (Tₘ), i.e. higher than 100°C, will be most advantageous.

Patent application WO2010041051 also describes crystalline hydrated form H of olaparib, which is obtained by dissolution of prepared solid solutions in water. No preparation method of form H applicable in the industrial scale is described therein.

Now, it has been surprisingly found out that a pharmaceutical preparation with controlled solubility and thus bioavailability can be prepared by using a suitable polymer with a low glass transition temperature (lower than 100°C) and/or a suitable crystalline form of olaparib.

### Disclosure of the Invention

The invention provides pharmaceutical preparations containing olaparib with controlled solubility and bioavailability of the active ingredient. The most stable and thus the least soluble crystalline form of active ingredients is often their hydrated form, usually in environments with a high activity of water, i.e. in aqueous solutions, or in environments with high relative air humidity. In the case of olaparib, it is Form H (the hydrate), which is produced by crystallization from oversaturated solutions of this active ingredient in water. Oversaturation, i.e. a higher than equilibrium concentration, can be generally achieved by using a thermodynamically unstable (e.g. amorphous) form of the given substance. An amorphous substance often tends to pass into a more stable crystalline form. The molecular arrangement of an amorphous solid can be represented as a "frozen liquid" having rheological properties of a solid.

A commonly used stabilization method of amorphous active ingredients is preparation of so-called molecular solid solutions or amorphous solid solutions, commonly designated as "solid solutions". In this invention, the term "solid solution" is used, which represents any solid composition composed of two components at minimum. In this case the solid solution comprises the pharmaceutically active ingredient (olaparib), which is dispersed in at least one component, e.g. in the polymer. The term "solid solution" as used herein means a dispersion of the component (olaparib) in a polymeric matrix. In some cases olaparib can be dispersed in the polymeric matrix in such manner that the components are immobilized in said matrix in their amorphous form. In the case where the components are dispersed at the molecular level, and such state is sometimes designated as a molecular solid solution, the resulting solid has a single glass transition temperature (Tg).

In the case where the component (olaparib) is dispersed in the polymeric matrix in the form of amorphous clusters, such state is sometimes designated as amorphous solid solution. The resulting amorphous solid solution has two or more glass transitions which belong respectively to the amorphous active pharmaceutical ingredient and to the given polymer or polymers.

Solid solutions can be prepared by dissolution of the active ingredient and a suitable polymer in a solvent and subsequent evaporation of the solvent, either by means of vacuum distillation or spray drying. Solid solutions can also be prepared by precipitation of a solution of the active ingredient and a suitable polymer by addition of a solvent in which both the substances are difficult to dissolve. Another preparation method of solid solutions is so-called hot-melt extrusion, i.e. melting of the active ingredient and a suitable polymer at an elevated temperature and subsequent cooling of the mixture. Polymers suitable for the preparation of solid solutions differ in their physicochemical characteristics such as solubility in water, glass transition temperature (T_{g}), viscosity and the like.

What has been surprisingly found out is that if suitable polymers with a low glass transition temperature, i.e. with T_{g} in the range from 40°C to 100°C, and suitable solubility in water are used, the release rate of olaparib into aqueous solutions can be controlled.

The invention provides a solid solution of olaparib with a hydrophilic polymer with the glass transition temperature in the range from 40°C to 100°C, preferably from 45°C to 70°C. In some embodiments, the hydrophilic polymer with the glass transition temperature from 40°C to 100°C is selected from the group consisting of Soluplus, which is chemically a polyvinyl caprolactam / polyvinyl acetate / polyethylene glycol copolymer, and Eudragits, which belong to copolymers of methacrylic acid esters. Out of Eudragits, Eudragit E100 and Eudragit E PO are especially preferred, which are chemically copolymers based on dimethyl aminoethyl methacrylate, butyl methacrylate and methyl methacrylate in the ratio of 2:1:1. In some embodiments, the olaparib:polymer ratio is in the range from 1:0.5 to 1:5, preferably from 1:1 to 1:3.

The invention further provides the use of the solid solution of olaparib with a hydrophilic polymer with a low glass transition temperature for the preparation of a pharmaceutical composition with controlled releasing of olaparib.

The invention further provides a pharmaceutical composition comprising the solid solution of olaparib with a hydrophilic polymer with a low glass transition temperature.

The invention further provides preparation of the solid solution of olaparib with a hydrophilic polymer with a low glass transition temperature by evaporation, spray drying or hot-melt extrusion.

The invention further provides a preparation method of olaparib Form H comprising dissolution of a crystalline or amorphous form of olaparib in an organic solvent with admixed water in the range from 0 to 50% and subsequent dilution of the resulting solution with water. In some embodiments, the organic solvent is a water-miscible organic solvent and the crystallization temperature is in the range from -20°C to 50°C, preferably - 5°C to 5°C. The water-miscible organic solvent can, e.g., be selected from the group consisting of methanol, ethanol, 2-propanol, acetone, dimethyl sulfoxide, N-methylpyrrolidone and dimethyl acetamide. In some embodiments, the crystalline or amorphous form of olaparib is stirred in a mixture of water and the organic solvent at a temperature in the range of -20°C to 50°C for 1 to 100 hours.

The invention further provides the use of form H, independently or in a mixture, for the preparation of a pharmaceutical composition with controlled releasing of olaparib.

The invention further provides olaparib hemisolvates, prepared from acetic acid, 1-propanol, 1-butanol and 2-butanol.

### Brief description of the Drawings

**Fig. 1****:** Comparison of solubility of the different crystalline forms of olaparib
**Fig. 2****:** X-ray pattern of crystalline olaparib Form H
**Fig. 3****:** X-ray pattern of isostructural solvate of olaparib
**Fig. 4****:** X-ray pattern of a molecular solid solution of olaparib and copovidone VA64 (1:2.3) prepared according to Example 5
**Fig. 5****:** X-ray pattern of a molecular solid solution of olaparib and Soluplus (1:2.3) prepared according to Example 6
**Fig. 6****:** X-ray pattern of an amorphous solid solution of olaparib and Eudragit E100 (1:2.3) prepared according to Example 7
**Fig. 7****:** X-ray pattern of an amorphous solid solution of olaparib and Eudragit EPO (1:2.3) prepared according to Example 9
**Fig. 8****:** X-ray pattern of a molecular solid solution of olaparib and copovidone VA64 (1:2.3) prepared according to Example 11

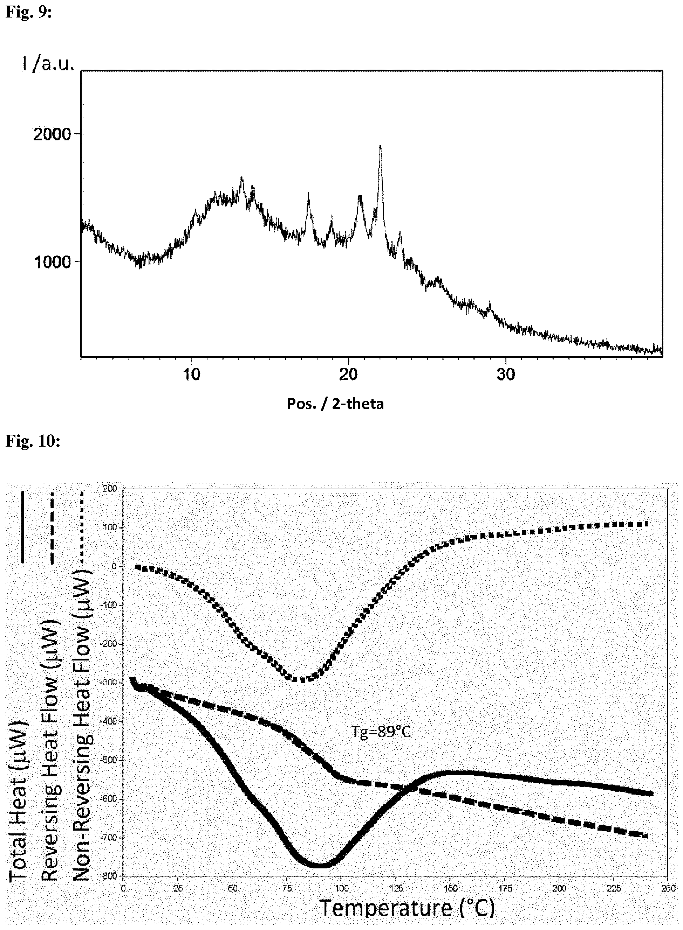

**Fig. 9****:** X-ray pattern of a molecular solid solution of olaparib and copovidone VA64 (1:2.3) prepared according to Example 11, exposed to 60°C/75% RH for 10 days
**Fig. 10****:** DSC record of a molecular solid solution of olaparib and copovidone VA64 (1:1) prepared according to Example 10

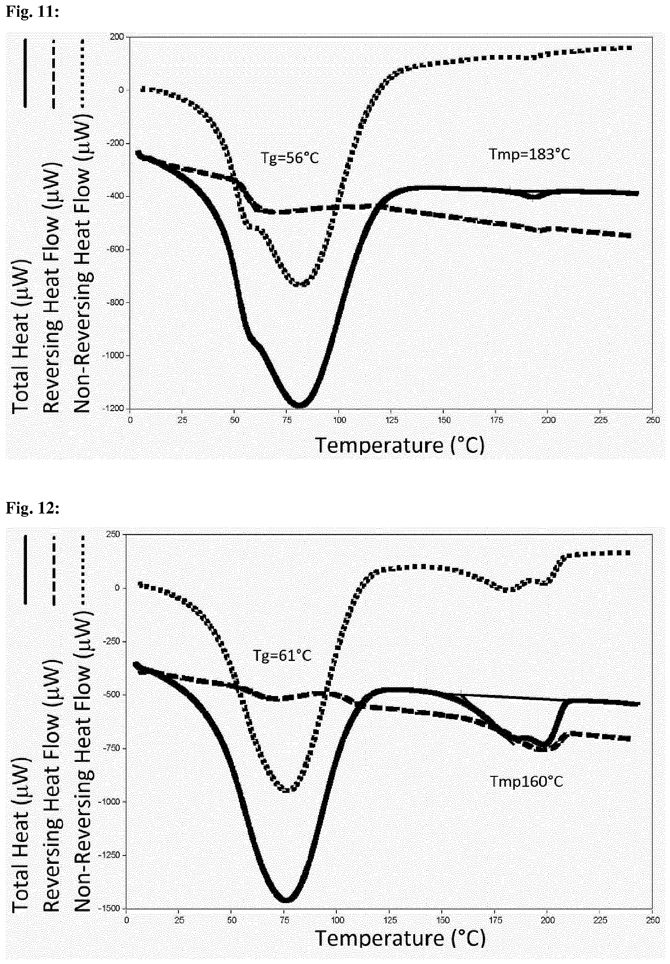

**Fig. 11****:** DSC record of a molecular solid solution of olaparib and copovidone VA64 (1:1) prepared according to Example 10, exposed to 40°C/75% RH for 10 days
**Fig. 12****:** DSC record of a molecular solid solution of olaparib and copovidone VA64 (1:1) prepared according to Example 10, exposed to 60°C/75% RH for 10 days

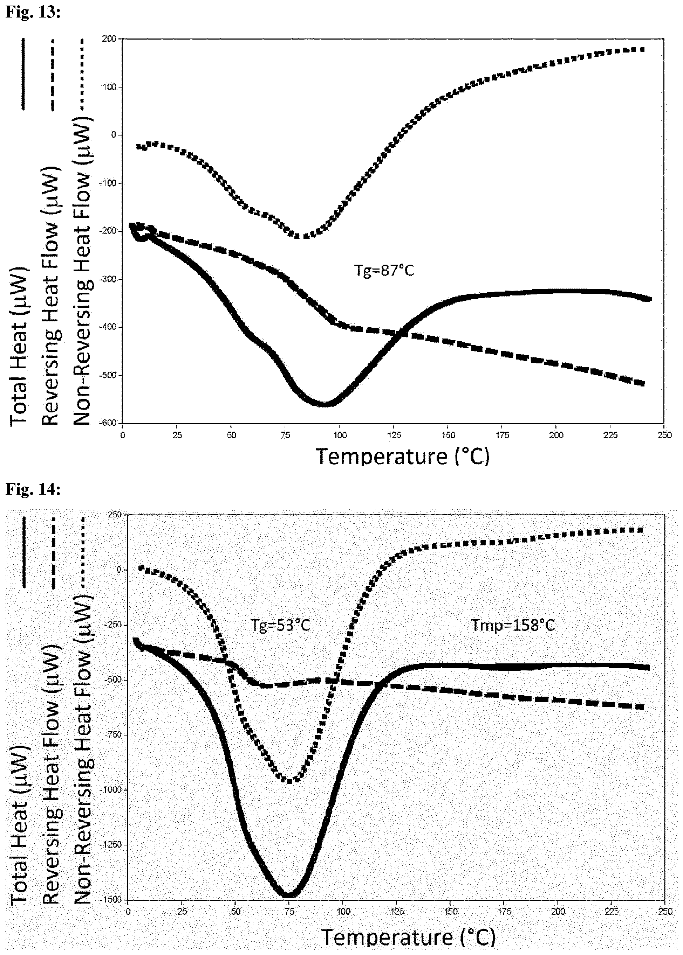

**Fig. 13****:** DSC record of a molecular solid solution of olaparib and copovidone VA64 (1:2.3) prepared according to Example 11
**Fig. 14****:** DSC record of a molecular solid solution of olaparib and copovidone VA64 (1:2.3) prepared according to Example 11, exposed to 40°C/75% RH for 10 days

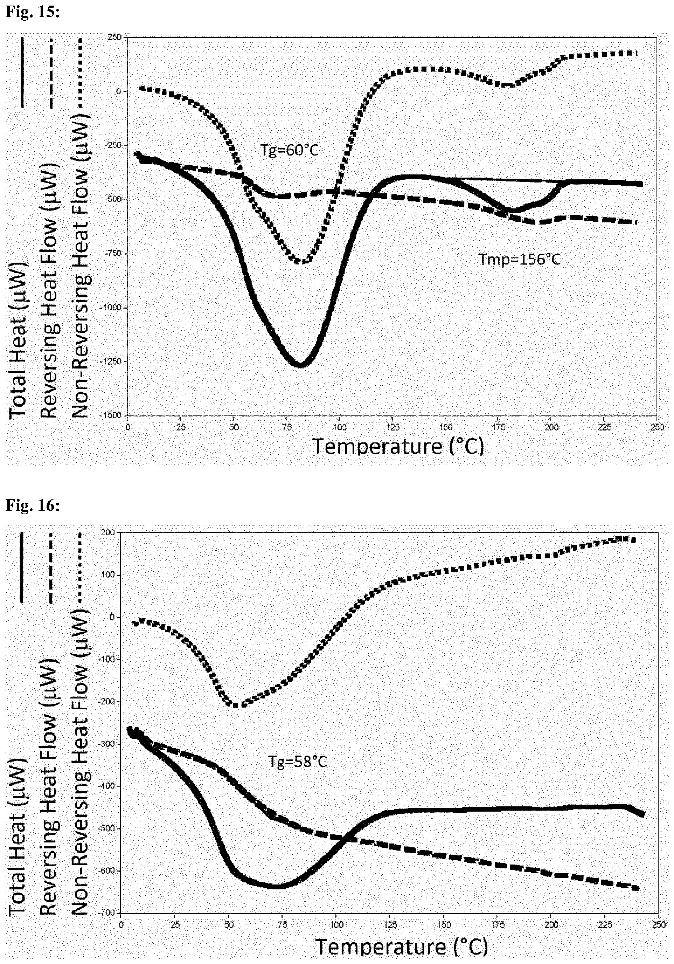

**Fig. 15****:** DSC record of a molecular solid solution of olaparib and copovidone VA64 (1:2.3) prepared according to Example 11, exposed to 60°C/75% RH for 10 days
**Fig. 16****:** DSC record of a molecular solid solution of olaparib and Soluplus (1:1) prepared according to Example 12

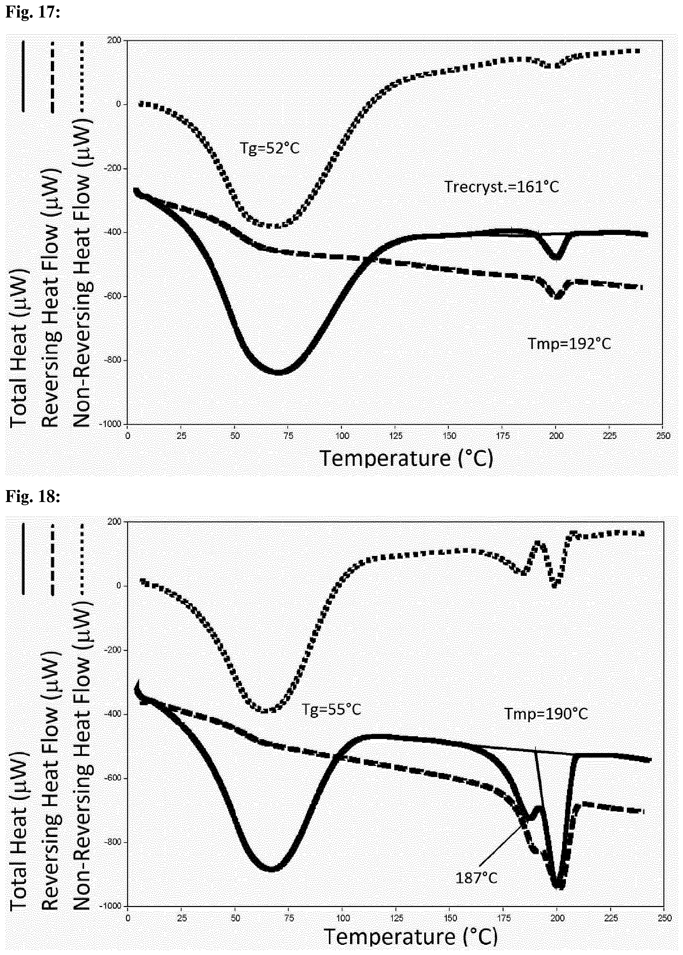

**Fig. 17****:** DSC record of a molecular solid solution of olaparib and Soluplus (1:1) prepared according to Example 12, exposed to 40°C/75% RH for 10 days
**Fig. 18****:** DSC record of a molecular solid solution of olaparib and Soluplus (1:1) prepared according to Example 12, exposed to 60°C/75% RH for 10 days

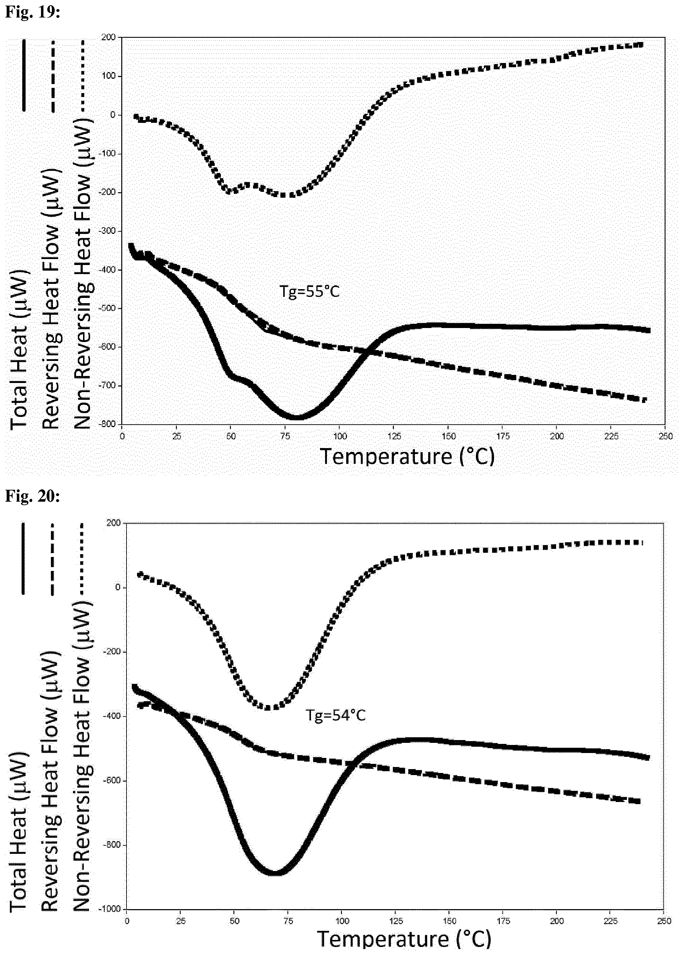

**Fig. 19****:** DSC record of a molecular solid solution of olaparib and Soluplus (1:3) prepared according to Example 13
**Fig. 20****:** DSC record of a molecular solid solution of olaparib and Soluplus (1:3) prepared according to Example 13, exposed to 40°C/75% RH for 10 days

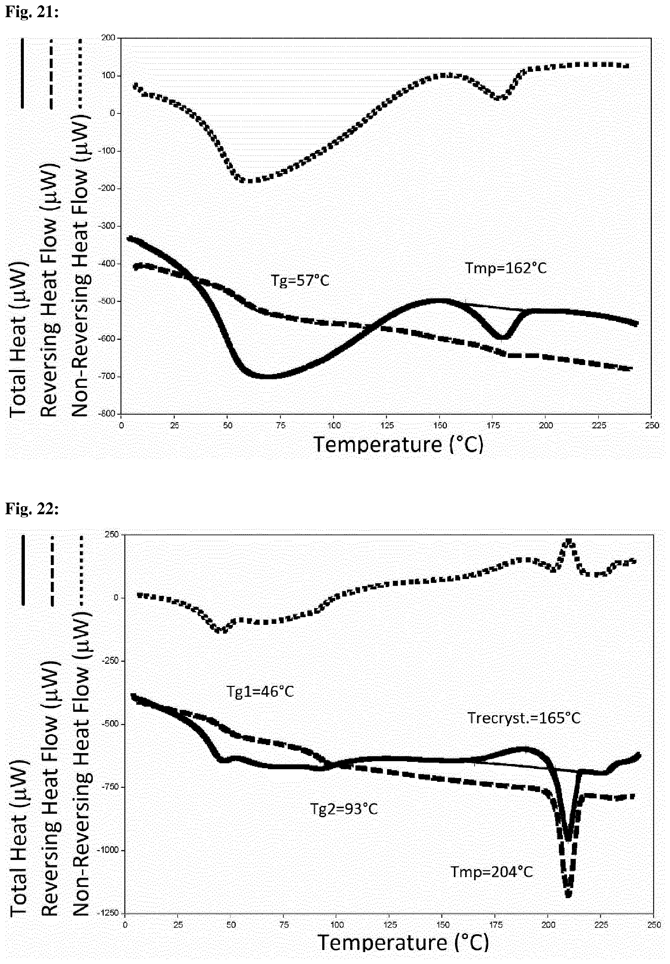

**Fig. 21****:** DSC record of a molecular solid solution of olaparib and Soluplus (1:3) prepared according to Example 13, exposed to 60°C/75% RH for 10 days
**Fig. 22****:** DSC record of an amorphous solid solution of olaparib and Eudragit E100 (1:1) prepared according to Example 14

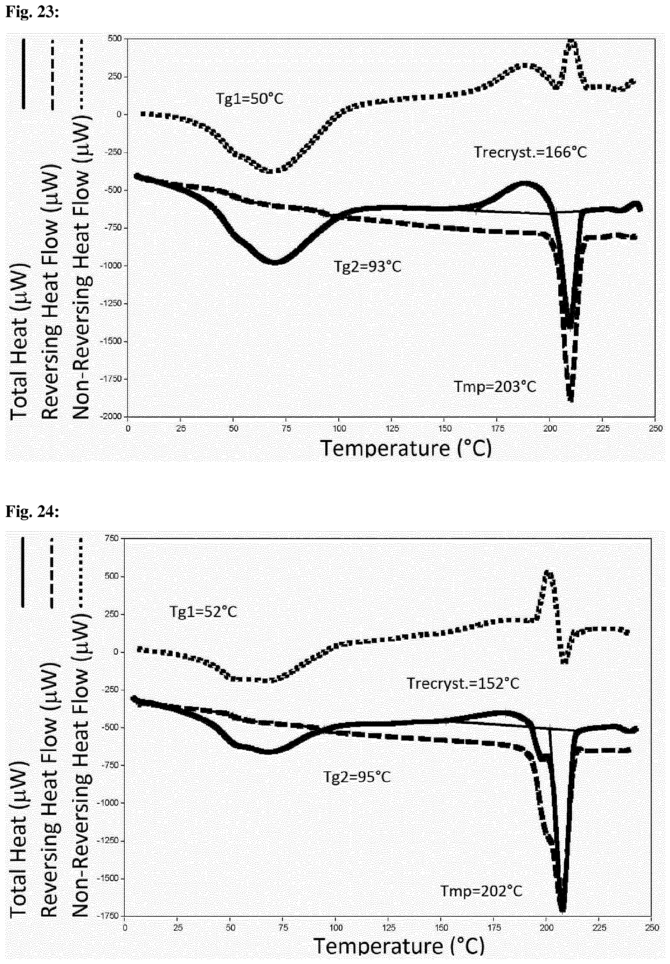

**Fig. 23****:** DSC record of an amorphous solid solution of olaparib and Eudragit E100 (1:1) prepared according to Example 14, exposed to 40°C/75% RH for 10 days
**Fig. 24****:** DSC record of an amorphous solid solution of olaparib and Eudragit E100 (1:1) prepared according to Example 14, exposed to 60°C/75% RH for 10 days

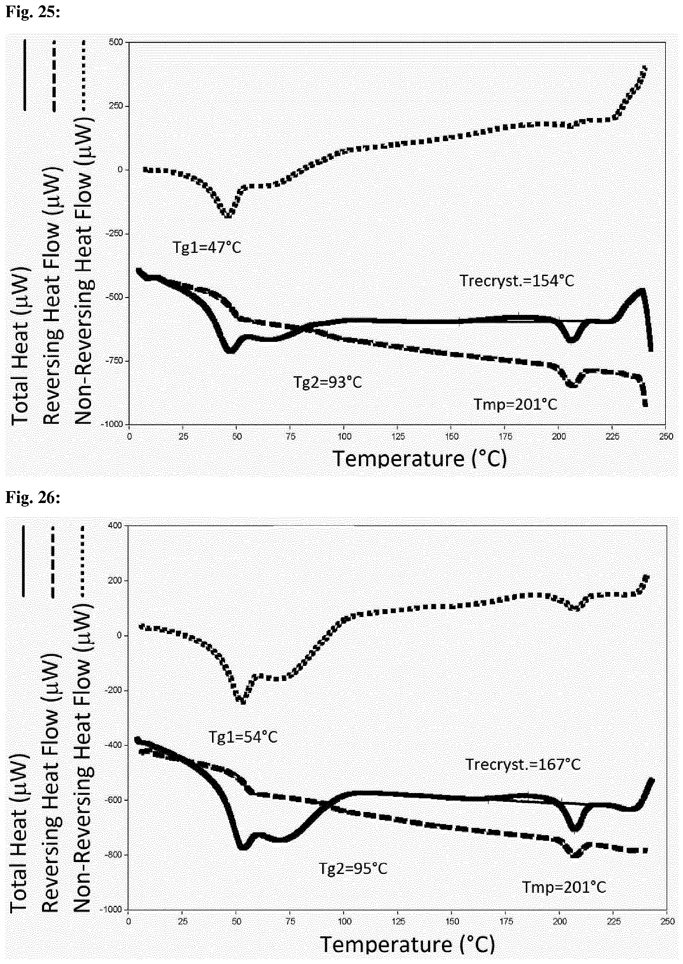

**Fig. 25****:** DSC record of an amorphous solid solution of olaparib and Eudragit E100 (1:3) prepared according to Example 15
**Fig. 26****:** DSC record of an amorphous solid solution of olaparib and Eudragit E100 (1:3) prepared according to Example 15, exposed to 40°C/75% RH for 10 days

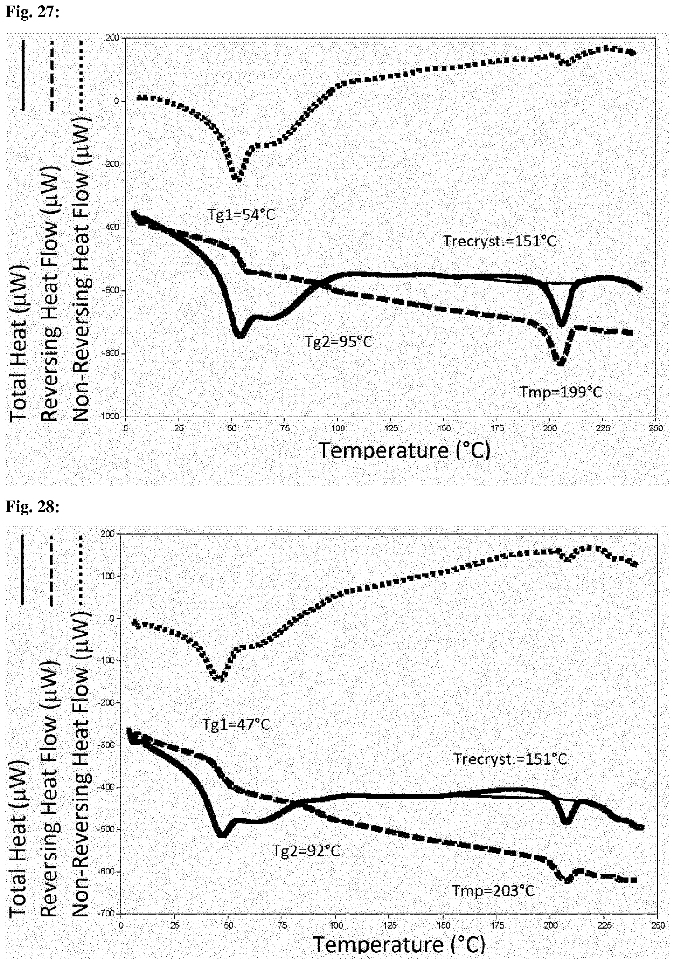

**Fig. 27****:** DSC record of an amorphous solid solution of olaparib and Eudragit E100 (1:3) prepared according to Example 15, exposed to 60°C/75% RH for 10 days
**Fig. 28****:** DSC record of an amorphous solid solution of olaparib and Eudragit EPO (1:3) prepared according to Example 16

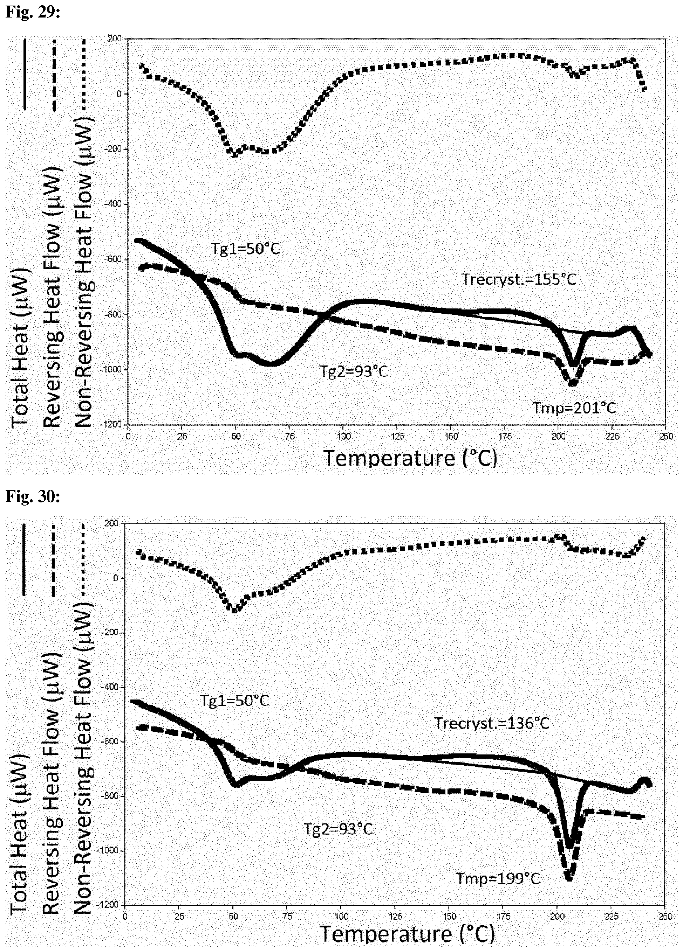

**Fig. 29****:** DSC record of an amorphous solid solution of olaparib and Eudragit EPO (1:3) prepared according to Example 16, exposed to 40°C/75% RH for 10 days
**Fig. 30****:** DSC record of an amorphous solid solution of olaparib and Eudragit EPO (1:3) prepared according to Example 16, exposed to 60°C/75% RH for 10 days **Fig. 31****:** Powder dissolution of solid solutions of olaparib in comparison with olaparib Form A
**Fig. 32****:** True dissolution of solid solutions of olaparib
**Fig. 33****:** Dynamic vapor sorption record of Eudragit E100
**Fig. 34****:** Dynamic vapor sorption record of Eudragit EPO
**Fig. 35****:** Dynamic vapor sorption record of Soluplus
**Fig. 36****:** Dynamic vapor sorption record of copovidone VA64

### Detailed description of the Invention

The invention provides solid solutions of olaparib with a hydrophilic polymer with the glass transition temperature in the range from 40°C to 100°C and their preparation. For the preparation of solid solutions of olaparib, the active ingredient in an amorphous or crystalline form (Form A, L, H or solvate) and a polymer with the glass transition temperature (Tg) in the range from 40°C to 100°C, preferably in the range from 45°C to 70°C, with good solubility in water can be used. Such polymers typically include substances containing in their structure polar hydrophilic (-OH, -CONH- etc.) or ionizable (-COOH, -NR₁R₂) groups, such as derivatives of polymethacrylate, copolymers based on polyvinyl caprolactam / polyvinyl acetate / polyethylene glycol and copolymers based on dimethyl aminoethyl methacrylate, butyl methacrylate and methyl methacrylate. Thus, an example of a suitable polymer can be e.g. Soluplus^{®} (T_{g} ∼ 70°C), Eudragit E100^{®} (T_{g} ∼ 45°C) or Eudragit E PO^{®} (T_{g} ∼ 45°C). These polymers can be used separately or in mixtures, or possibly with the use of an addition of a surfactant and/or plasticizer (e.g. polyethylene glycol, sodium dodecyl sulfate etc.). The olaparib:polymer ratio can vary in the range roughly frpm 1:0.5 to 1:5, preferably from 1:1 to 1:3.

Solid solutions can be prepared in various ways. One of the procedures comprises dissolution of olaparib (Form A, L, H, a solvate or amorphous form of olaparib) and the polymer in a suitable solvent and subsequent evaporation of the solvent. An organic solvent, water or mixtures of various solvents can be used for the dissolution. As a suitable organic solvent e.g. methanol, ethanol, 2-propanol, dichloromethane, acetone etc. can be mentioned. The solvent is then evaporated at a temperature in the range from 20°C to the boiling point of the solvent at the atmospheric or reduced pressure. For example, a rotary vacuum evaporator, fluid granulator, spray drier etc. can be used for the evaporation of the solvent.

Other options of preparation of solid solutions include procedures without the use of a solvent. In these procedures, olaparib is mixed with a polymer and the mixture is heated up, providing a melt. Commonly, a temperature in the range of 20°C to 40°C above T_{g} of the selected polymer is used. Thus, a temperature in the range roughly from 60°C to 190°C, preferably from 90°C to 160°C can be used. The melt is subsequently cooled down, providing an amorphous solid substance. As some examples of these processes hot melt extrusion, hot melt granulation, high shear mixer etc. can be mentioned.

The selected polymers can be mixed with olaparib in concentrations of API : the polymer with glass transition (T_{g}) value in the range of from 40°C to 100°C in a ratio in the range of from 1:0.5 to 1:5, preferably from 1:1 to 1:3.

The invention further provides preparation of olaparib Form H and its use for the preparation of a drug form with controlled releasing of the active ingredient. Form H can be prepared by dissolution of olaparib in a suitable water-miscible solvent such as alcohols (methanol, ethanol and the like), ketones (acetone), cyclic ethers (tetrahydrofuran, dioxane) or polar aprotic solvents (dimethyl sulfoxide, dimethyl acetamide and the like), followed by dilution with water having a low temperature. Water can be added to the solution of olaparib or conversely the solution of olaparib can be added to water. The crystallization temperature should be in the range from -20°C to about 50°C, preferably -5°C to 5°C. To maintain a temperature close to 0°C, water can also be in a mixture with ice.

The invention further provides hemisolvates of olaparib, prepared from acetic acid, 1-propanol, 1-butanol and 2-butanol (the molar ration of API : solvent is 1:0.5), obtained from a suspension or solution.

A drug form (tablet, capsule) can be prepared by mixing of olaparib Form H (hydrate), or another solvate (acetone, ethanol, ethyl acetate, 2-propanol, acetic acid, 1-propanol, 1-butanol, 2-butanol, etc.) and/or solid solution of olaparib with a hydrophilic polymer with the glass transition temperature in the range from 40°C to 100°C and possibly with other excipients (fillers, disintegrants, surfactants and the like) with the use of standard pharmaceutical technologies (direct tabletting, dry or wet granulation, filling capsules with powder and the like). The particle size of the selected form of olaparib (crystalline hydrate/solvate or amorphous solid solution of the active ingredient) can be modified (reduced) to the desired value, i.e. in the range roughly from 1 µm to 1.5 mm, preferably from 10 µm to 1000 µm.

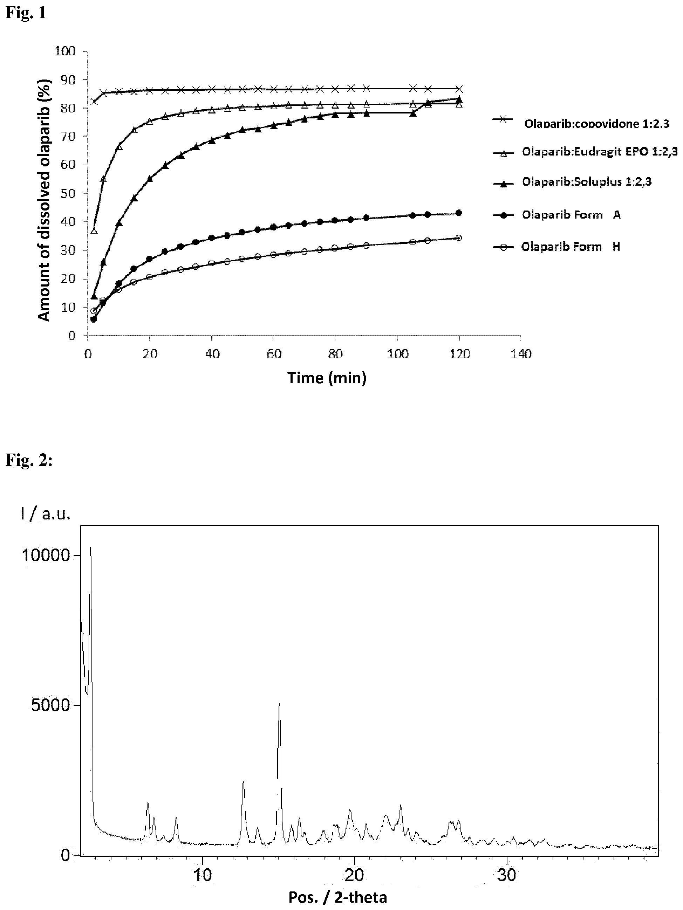

An advantage of the approach in accordance with the present invention is easy preparation of the said forms of olaparib as well as subsequent preparation of the drug form. A suitable selection of a form of olaparib, or mixtures of its forms (hydrate, solvate, solid solution) can be used to achieve the required release of the active ingredient into an aqueous solution as can be seen in Figs. 1, 31 and 32.

### Experimental part

*X-ray measurement parameters:* The diffraction patterns were measured using an X'PERT PRO MPD PANalytical diffractometer, used radiation CuKα (λ=1.542 Å), excitation voltage: 45 kV, anode current: 40 mA, measured range: 2-40° 2θ, increment: 0.02° 2θ. For the measurement a flat powder sample was used that was placed on a Si plate. For the setting of the primary optical equipment programmable divergence slits with the irradiated area of the sample of 10 mm, 0.02 rad Soller slits and a 1/4° anti-diffusion slit were used. For the setting of the secondary optical equipment an X'Celerator detector with maximum opening of the detection slot, 0.02 rad Soller slits and a 5.0 mm anti-diffusion slit were used.

The *dissolution* (*dissolution rate*) was measured using a Sotax AT-7 dissolution device. The dissolution was carried out in 150 ml of a pH 2.0 solution (10mM HCl) at the constant stirring rate of 125 rpm. Samples were extracted at 5-minute intervals and the concentration of dissolved olaparib was measured with a Jena Analytik 200 UV/Vis spectrofotometer at the wavelength of 263 nm with the use of 5 mm cuvettes.

*Differential scanning calorimetry* (*DSC*) records were measured using a Discovery DSC device from TA Instruments. Charge of the sample into a standard A1 crucible (40 µL) was between 3-5 mg and the heating rate was 5°C/min. The temperature program used consisted in 1 stabilization minute at 0°C and then heating to 250°C at a heating rate of 5°C/min (amplitude = 0.8°C and period = 60 s). 5.0 N₂ at a flow rate of 50 ml/min was used as the carrier gas.

*True dissolution:* Discs were prepared by compression of 20 mg of a solution of drug /solid into a disc under the pressure of 100 kg for 120 s using a screw press (Sirius Analytical Instr. Ltd., Forest Row, UK). The exposed surface of the resulting disc was 0.07 cm². The dissolution test was carried out at the temperature maintained at 37°C. Assessment of the released amount of the drug was carried out on the Sirius inForm platform (Sirius Analytical Instr. Ltd., Forest Row, UK) with current assessment of the pH. Each dissolution vessel contained 40 ml of an aqueous medium maintained at the constant pH of 2 (10 mM, HCl + 0.2% SLS buffer). The disc (matrix) holder was immersed in said dissolution medium and rotated at 100 rpm. The amount in the solution was determined by means of multi-wavelength UV absorption using an *in situ* probe. The spectra were recorded every 30 seconds.

*Powder dissolution*: A dissolution device (Sotax AT-7) was used for measuring powder dissolution, fitted with minipaddles, which were rotated at 125 rpm during the experiment for 105 minutes; then the rate was increased to 150 rpm up to the end of the experiment at minute 120. 150 ml of a 10 mM solution of hydrochloric acid at pH 2 was selected as the dissolution medium. This medium was tempered at 37°C during the experiment. Samples were taken at regular intervals and the concentration of the dissolved olaparib was determined by means of the Jena Analytik Specord 200 plus UV/Vis spectrophotometer at the wavelength of 263 nm using 5 mm cuvettes.

*Dynamic vapor sorption* (*DVS*) was measured using a DVS Advantage 1 device from Surface Measurement Systems. Charge of the sample into a quartz bowl was between 15-30 mg and the temperature in the device is between 25-25.2°C. The measuring program used: the sample was exposed to two cycles (Soluplus) or one cycle (Eudragit and Copovidone VA64) which have development from the relative humidity of 0% up to 90% RH (sorption) and then from 90% to 0% RH (desorption) with a 10% RH step. This development was repeated in the second cycle (Soluplus). 4.0 N₂ at a flow rate of 200 sccm was used as the carrier gas.

*Stress stability test*: the prepared compositions of ground extrudates form Examples 10-16 were exposed in stability chambers at the conditions of 40°C and 60°C at 75% relative air humidity (RH) in opne glass vials for 10 days.

### Examples

The working examples below are only provided to illustrate and to explain the invention and are not in any case intended to restrict the protection scope, which is only delimited by the wording of the claims.

Olaparib form A was prepared according to the procedure described in WO2008047082, Example 1.

### Example 1

### Olaparib Form H

Olaparib (100 mg) was dissolved at 65°C in a methanol:water mixture, 2:1 (2 ml). Then, the solution was injected into an intensively stirred mixture of water and ice (15 ml and 5 g). The mixture was stirred for 1 h at 4°C. After slow heating up to 25°C the product was filtered off, washed with water and dried (30°C, 120 mbar, 8 hours). XRPD analysis: Form H (Fig. 2).

### Example 2

### Olaparib Form H

Olaparib hemibutanone solvate (50 mg), prepared by crystallization of olaparib Form A from butanone, was stirred in a mixture with water at 25°C for two days. Then, the suspension was filtered and the product was dried. XRPD analysis: Form H (Fig. 2).

### Example 3a

### Olaparib.acetic acid hemisolvate

200 mg of olaparib Form A was suspended in 0.25 ml of acetic acid at 50°C. The suspension was kept in a closed vessel at 50°C under continuous stirring for 72 hrs.

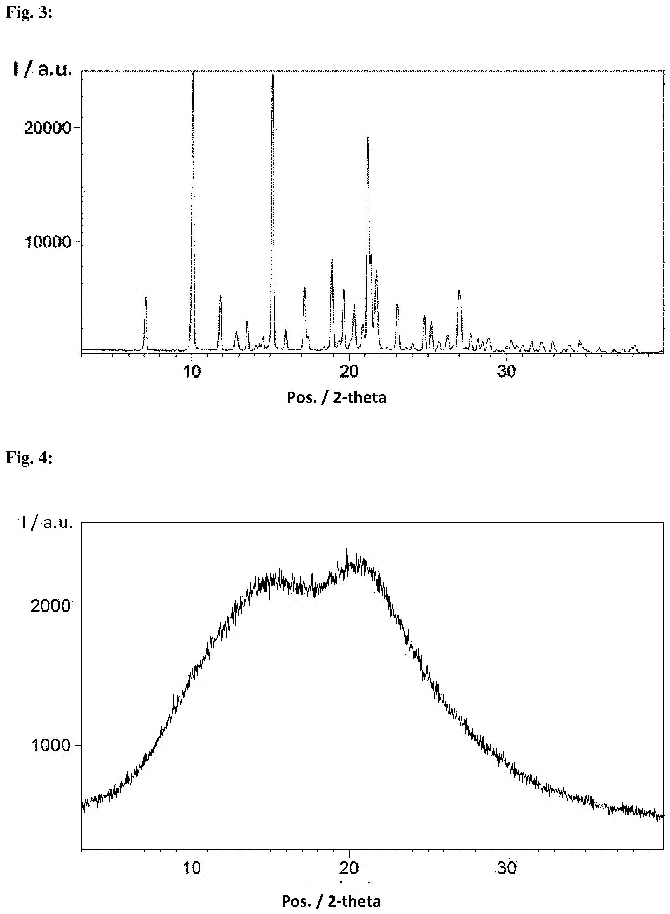

The solid was obtained by filtration and dried by vacuum suction at 40°C. XRPD analysis: isostructural solvate (Fig. 3).

### Example 3b

### Olaparib.1-propanol hemisolvate

200 mg of olaparib Form A was suspended in 0.25 ml of 1-propanol at 50°C. The suspension was kept in a closed vessel at 50°C under continuous stirring for 72 hrs.

The solid was obtained by filtration and dried by vacuum suction at 40°C. XRPD analysis: isostructural solvate (Fig. 3).

### Example 3c

### Olaparib.1-butanol hemisolvate

200 mg of olaparib Form A was suspended in 0.25 ml of 1-butanol at 50°C. The suspension was kept in a closed vessel at 50°C under continuous stirring for 72 hrs.

The solid was obtained by filtration and dried by vacuum suction at 40°C. XRPD analysis: isostructural solvate (Fig. 3).

### Example 3d

### Olaparib.2-butanol hemisolvate

200 mg of olaparib Form A was suspended in 0.25 ml of 2-butanol at 50°C. The suspension was kept in a closed vessel at 50°C under continuous stirring for 72 hrs.

The solid was obtained by filtration and dried by vacuum suction at 40°C. XRPD analysis: isostructural solvate (Fig. 3).

### Example 4a

### Olaparib.2-propanol solvate

50 mg of olaparib (Form A) was dissolved in 2-propanol (2 ml) under reflux. The solution was gradually evaporated to dryness. The evaporation residue was dried under reduced pressure (200 mbar) at 40°C for 12 hrs.

### Example 4b

### Olaparib.acetic acid solvate

50 mg of olaparib (Form A) was dissolved in 0.1 ml of acetic acid at 50°C under continuous stirring. Then olaparib.2-propanol solvate was added and the solution was further agitated at 50°C/750 rpm for 2 weeks. The solid was obtained by filtration and dried by vacuum suction at 40°C. XRPD analysis: isostructural solvate (Fig. 3).

### Example 5

### Olaparib:copovidone VA64 solid solution (1:2.3)

Olaparib (300 mg) and copovidone (690 mg) were dissolved in an ethanol:water mixture, 9:1 (4.2 ml) at 70°C. The solution was then evaporated in a rotary vacuum evaporator at 40°C. The evaporation residue was dried at a reduced pressure (120 mbar) at the temperature of 40°C for 12 h. XRPD: amorphous form (Fig. 4).

### Example 6

### Olaparib:Soluplus solid solution (1:2.3)

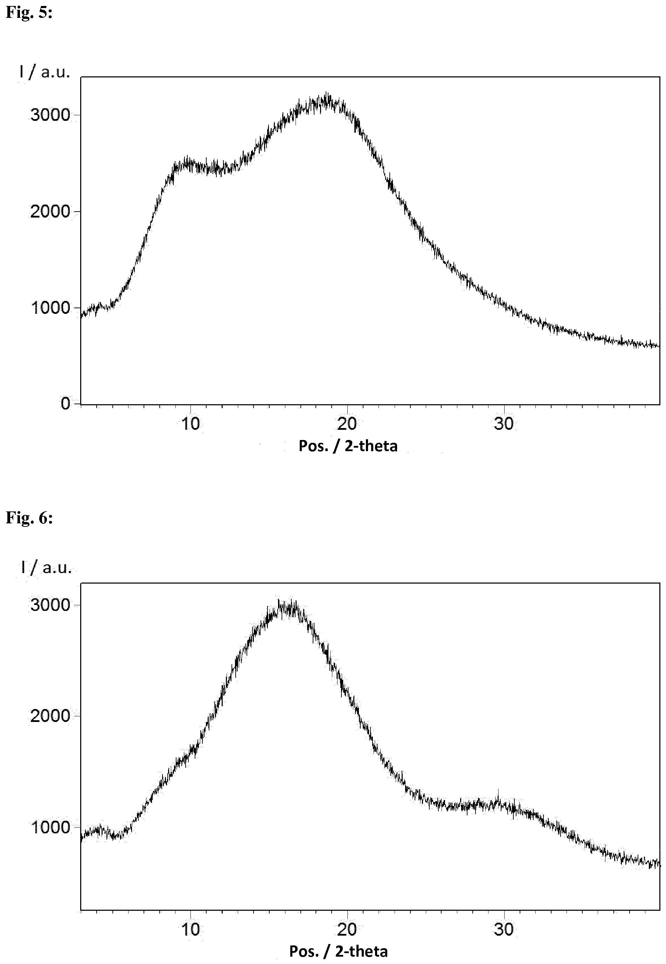

Olaparib (300 mg) and Soluplus (690 mg) were dissolved in an ethanol:water mixture, 9:1 (4.1 ml) at 70°C. The solution was then evaporated in a rotary vacuum evaporator at 40°C. The evaporation residue was dried at a reduced pressure (120 mbar) at the temperature of 40°C for 12 h. XRPD: amorphous form (Fig. 5).

### Example 7

### Olaparib:Eudragit E100 solid solution (1:2.3)

Olaparib (300 mg) and Eudragit E100 (690 mg) were dissolved in an ethanol:water mixture, 9:1 (4.2 ml) at 70°C. The solution was then evaporated in a rotary vacuum evaporator at 40°C. The evaporation residue was dried at a reduced pressure (120 mbar) at the temperature of 40°C for 12 h. XRPD: amorphous form (Fig. 6)

### Example 8

### Olaparib:Eudragit E100 solid solution (1:2.3)

Olaparib (300 mg) and Eudragit E100 (690 mg) were dissolved in an ethanol:water mixture, 9:1 (4.2 ml) at 70°C. The solution was then cooled down to 20°C and added to *tert*-butyl methyl ether (30 ml) cooled to 0°C, being stirred. The product was filtered off and dried at a reduced pressure (120 mbar) at the temperature of 40°C for 12 h. XRPD: amorphous form (Fig. 6).

### Example 9

### Olaparib:Eudragit E PO solid solution (1:2.3)

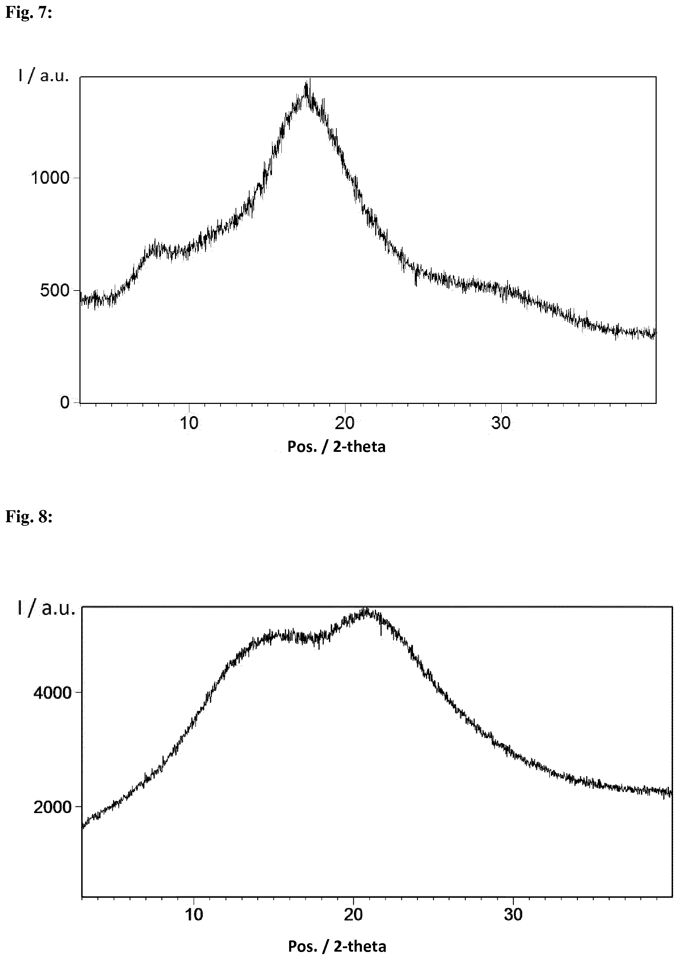

Olaparib (300 mg) and Eudragit E PO (690 mg) were dissolved in an ethanol:water mixture, 9:1 (4.2 ml) at 70°C. The solution was then evaporated in a rotary vacuum evaporator at 40°C. The evaporation product was dried at a reduced pressure (120 mbar) at the temperature of 40°C for 12 h. XRPD: amorphous form (Fig. 7).

### Examples 10-16

### Solid solutions prepared by hot-melt extrusion (HME)

The following samples were prepared as physical mixture of API:polymer in amounts given in Table 1 below. The mixtures were subsequently dosed into a hot-melt extruder, wherein they were further blended at the temperatures indicated in the table.

**Table 1: Composition and processing parameters for Examples 10 - 16**

| Example | Composition | Amount of mixed components | Dosing rate of the blend | Temperatures of various HME zones |
|---|---|---|---|---|
| 10 | Olaparib:Copovidone VA64 1:1 | 2.5g polymer-2.5 g API | 6% | 130, 180, 200, 195°C |
| 11 | Olaparib:Copovidone VA64 1:2.3 | 3.485g polymer-1.515g API | 6% | 130, 180, 200, 195°C |
| 12 | Olaparib: Soluplus 1:1 | 2.5g polymer-2.5 g API | 8% | 125, 195, 200, 160°C |
| 13 | Olaparib: Soluplus 1:3 | 3.75g polymer-1.25g API | 8% | 140, 180, 180, 170°C |
| 14 | Olaparib:Eudragit E100 1:1 | 2.5g polymer-2.5 g API | 5% | 120,200,210, 170°C |
| 15 | Olaparib:Eudragit E100 1:3 | 3.75g polymer-1.25g API | 8% | 120, 150, 150, 140°C |
| 16 | Olaparib:Eudragit EPO 1:3 | 3.75g polymer - 1.25g API | 8% | 120, 150, 150, 140°C |

| | | | | |
|---|---|---|---|---|
| Note: - The reason why the composition API:Eudragit EPO (1:1) was not taken into the assessment is that this composition does not have both satisfactory physical stability during stress stability assessment and optimal processability in hot-melt extrusion (necessity of briquetting the blend before compaction). - For improving flow ability, the mixture of Olaparib and Eudragitu EPO 1:3 was compacted by means of briquetting before the extrusion. | | | | |

### Example 17

### Stress Stability Assessment

Actual physical stability of the solid solution was measured, after the steress test, by modulated DSC instead of the originally used measurement of powder X-ray pattern. The reason is that X-ray has significantly higher detection limit, which corresponds approximately to 4 J/g of endotherm in modulated DSC. For this reason the modulated DSC was preferred to the X-ray for assessment of the mixtures prepared by hot-melt extrusion.

The residual water contained in the polymer has a fundamental influence on physical and chemical properties of amorphous solid solutions, which was confirmed in stress stability assessment. Water acts as a plasticizer and causes important increase of molecular mobility, which lowers the Tg and increases the rate of crystallization. At high temperatures and high relative humidity (RH) it is expected that it accelerates any interaction between the drugs and the polymers. Therefore, it is important to monitor Tg in the conditions with elevated humidity.

It is clear from Table 2 that Tg in amorphous solid solutions of Eudragit E100 (both 1:1 and 1:3) and EPO (1:3) does not drop even in the conditions of 60°C/75% RH, while Tg of copovidone (1:2.3) dropped from 86.5°C to 53°C already in the conditions of 40°C/75% RH. The mixture of API:copovidone (1:2.3) shows a crystalline peak detectable even already in the X-ray pattern, associated with crystalline Form A of olaparib, in the conditions of 60°C/75% RH, while the solid solutions prepared from Eudragit E100 and EPO remained unchanged in both these conditions. Soluplus in the ratio of 1:3 shows a higher stability than both the tested mixtures with copovidone in the conditions of 40°C/75% RH. For a better idea of the extent of crystallinity, an X-ray powder pattern was measured for the composition of API:copovidone 1:2.3.

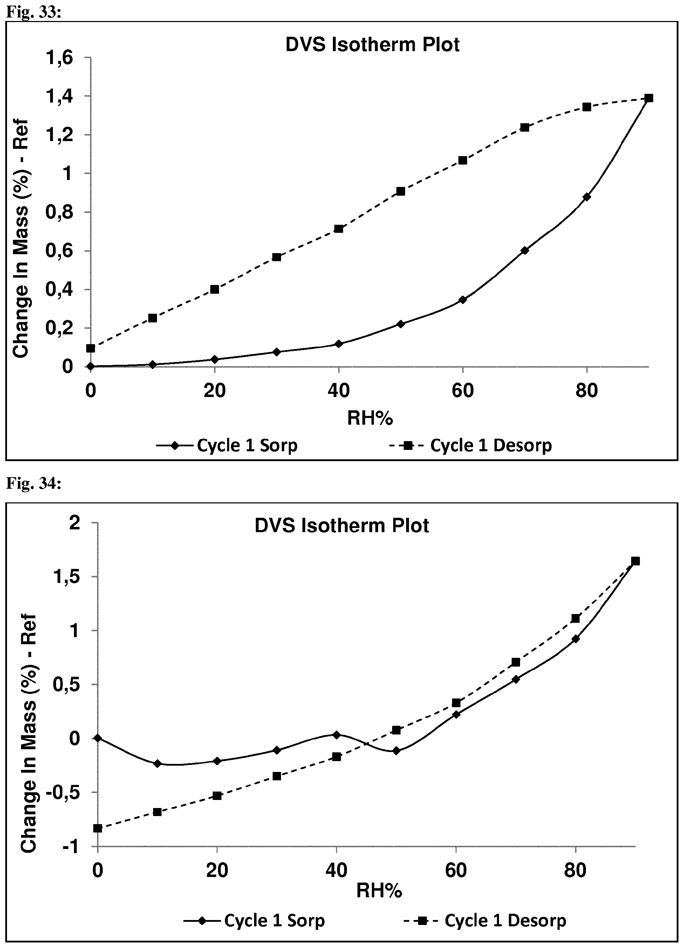

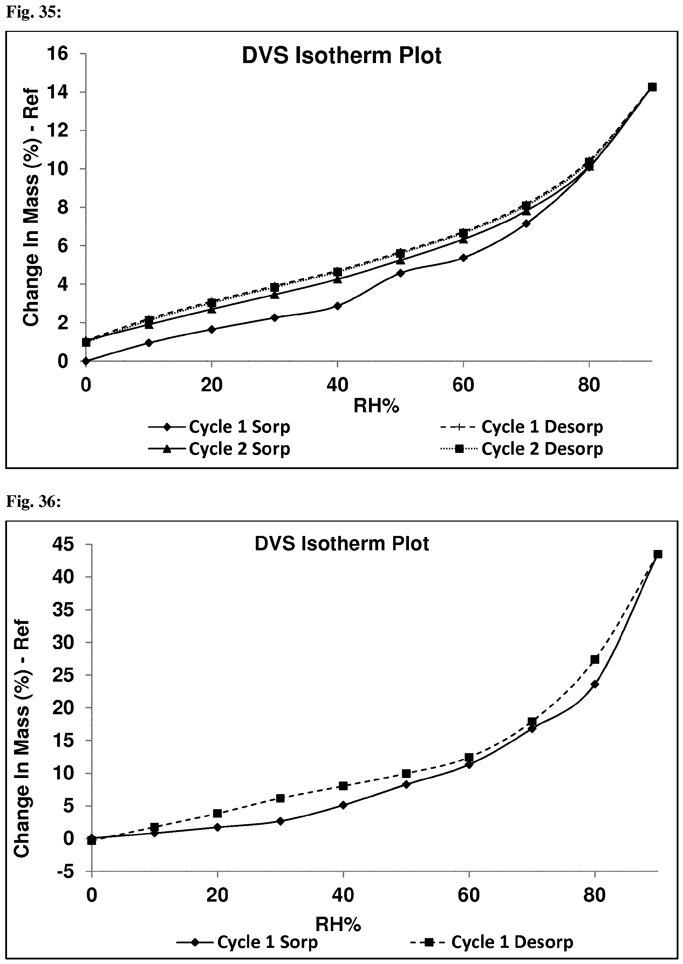

This phenomenon can be explained by the fact that copovidone is remarkably more hygroscopic in comparison with all tested polymers having low glass transition temperature. This high amount of water present associated with the plastifying effect can lead to formation of crystallization nuclei in the course of stability testing. By comparing the sorption isotherms (Figs. 33-36) of Soluplus and of copovidone it can be seen that Soluplus absorbs about half of moisture than copovidone and absorption of humidity by Eudragit E100 and EPO is in total one order lower. The prepared solutions of olaparib with Soluplus, Eudragit E100 and EPO show hygroscopicity at 50% RH at the level <6%, while solid solutions with copovidone at the same conditions show hygroscopicity of approximately 10%.

It follows from the data obtained that the greater ability of Eudragit E100 and EPO to stabilize the amorphous solution of olaparib is not caused by better miscibility with these excipients as compared to copovidone since copovidone forms a molecular solid solution, while Eudragit E100 and EPO form a dispersion of clusters of amorphous olaparib in the solid polymer (amorphous solid solution). Accordingly, the improved physical stability can be attributed to the lower hygroscopicity of these polymers.

**Table 2: Results of stress stability assessment**

| **Composition** | **40°C 75% RH** | **60°C 75% RH** |
|---|---|---|
| **Copovidone VA64 1:1** | Partial crystalline admixture detected by DSC | Partial crystalline admixture detected by DSC and XRPD |
| **Tg=89°C** | Tg=56°C | Tg=61°C |
| **Copovidone VA64 1:2.3** | Partial crystalline admixture detected by DSC | Partial crystalline admixture detected by DSC and XRPD |
| **Tg=87°C** | Tg=53°C | Tg=60°C |
| **Soluplus 1:1** | Partial crystalline admixture detected by DSC | Partial crystalline admixture detected by DSC and XRPD |
| **Tg=58°C** | Tg=52°C | Tg=55°C |
| **Soluplus 1:3** | Molecular solid solution | Partial crystalline admixture detected by DSC and XRPD |
| **Tg=55°C** | Tg=54°C | Tg=57°C |
| **Eudragit E100 1:1** | Amorphous solid solution | Amorphous solid solution |
| **Tg=46°C;93°C** | Tg=50°C;93°C | Tg=52°C;95°C |
| **Eudragit E100 1:3** | Amorphous solid solution | Amorphous solid solution |
| **Tg=47°C;93°C** | Tg=54°C;95°C | Tg=54°C;95°C |
| **Eudragit EPO 1:3** | Amorphous solid solution | Amorphous solid solution |
| **Tg=47°C;92°C** | Tg=50°C;93°C | Tg=50°C;93°C |

### Example 18

### Testing of olaparib dissolution

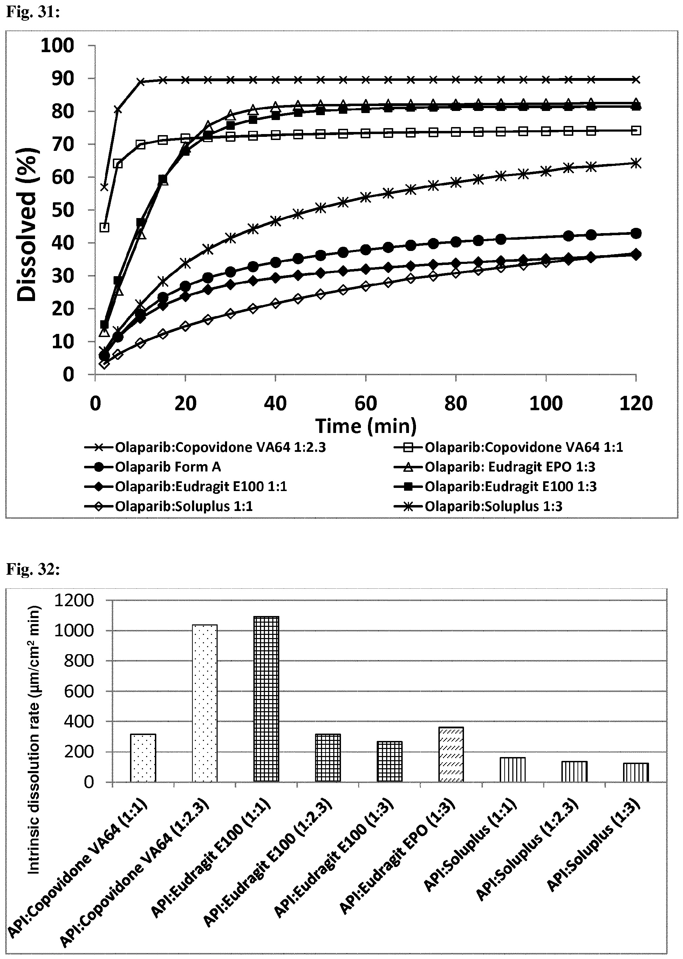

For testing of dissolution of the drug from the mixture, methods of powder and true dissolution were used. In the powder dissolution (Fig. 31) the defined amount of the extrudate API:polymer is charged into the container of the dissolution device at the defined time and the amount of olaparib released in time is determined. On the other hand, in the method of true dissolution (Fig. 32), discs were first prepared by compression of olaparib or of the tested solid solution into the disc cavity at a defined pressure for a defined time. Dissolution of the drug is in this case assessed only from the given defined surface. This fact has the consequence that with the increasing amount of the polymer (for examples, ratios of API:Eudragit E100 1:3 and API:Soluplus 1:3) the amount of the drug released in time decreases. This trend is not observed in the sample of API:copovidone 1:2.3 since this, compared to the two above-mentioned polymers, is a markedly faster hydrating polymer, which quickly erodes in true dissolution, which results in releasing olaparib into the solution with a faster kinetics.

By assessing the release profiles by the method of true dissolution (Fig. 32) it has been surprisingly found out that the composition of API:Eudragit E100 in the ratio of 1:1 provides a comparable amount of the drug in time as the reference composition of API:copovidone 1:2.3. the lower ratio of the API and the polymer can be considered as a fundamentally more advantageous solution since it secures higher safety of the preparation in terms of the amount of the polymer administered in a single dose, as well as a lower size of the resulting dosage form, which favorably influences patient's adherence to the treatment.

The data from powder dissolution (Fig. 31), as opposed to true dissolution, show release profiles from the prepared compositions by a method standardly used for *in-vitro* assessment of medicinal products. It can be read from these data that the composition API:Eudragit EPO and API:Eudragit E100 in the ratio 1:3 show comparable release profiles with that of the reference composition API:copovidone 1: 2.3. The compositions of API:polymer 1:1 are burdened with lower wettability rate of the prepared extrudate, which, of course, is a phenomenon which can be compensated by addition of a surfactant in the subsequent steps of preparing of the pharmaceutical composition (granulate, tablets, capsules).

In general, the two dissolution methods are not mutually comparable since they are based on different principles. The differenced in the profiles of the individual compared compositions are augmented by the absence of the surfactant in the dissolution medium in the powder dissolution.

## Claims

1. A solid solution of olaparib, **characterized in that** it comprises a hydrophilic polymer with the glass transition temperature in the range from 40°C to 100°C.

2. The solid solution of olaparib according to claim 1, wherein the hydrophilic polymer with the glass transition temperature in the range from 40°C to 100°C is selected from the group consisting of a polyvinyl caprolactam / polyvinyl acetate /polyethylene glycol copolymer and copolymers based on dimethyl aminoethyl methacrylate, butyl methacrylate and methyl methacrylate.

3. The solid solution of olaparib according to claim 2, wherein the ratio of the constituents of the copolymer based on dimethyl aminoethyl methacrylate, butyl methacrylate and methyl methacrylate is 2:1:1.

4. The solid solution of olaparib according to claims 1 to 3, wherein the olaparib:polymer ratio is in the range from 1.0.5 to 1.5, preferably from 1:1 to 1:3.

5. Use of the solid solution of olaparib according to claims 1 to 4 for the preparation of a pharmaceutical composition with controlled releasing.

6. A pharmaceutical composition, comprising the solid solution of olaparib according to claims 1 to 4.

7. The pharmaceutical composition according to claim 6, **characterized in that** it further contains a surfactant and/or plasticizer.

8. A method for preparing the solid solution of olaparib according to claims 1 to 4, **characterized in that** it comprises dissolution of olaparib and said polymer in a suitable solvent selected from the group consisting of methanol, ethanol, 2-propanol, dichloromethane, acetone, water and their mixtures, followed by removal of the solvent.

9. A method for preparing the solid solution of olaparib according to claims 1 to 4, **characterized in that** it comprises mixing of olaparib with said polymer, and subsequent heating of this mixture, providing a melt.

10. A method for preparing olaparib Form H, **characterized in that** it comprises dissolution of a crystalline or amorphous form of olaparib in an organic solvent with admixed water in the range from 0 to 50% and subsequent dilution of the obtained solution with water.

11. The method for preparing olaparib Form H according to claim 10, **characterized in that** the organic solvent is a water-miscible organic solvent and the temperature is in the range of -20°C to 50°C, preferably -5°C to 5°C.

12. The method for preparing olaparib Form H according to claim 11, **characterized in that** the water-miscible organic solvent is selected from the group consisting of methanol, ethanol, 2-propanol, acetone, dimethyl sulfoxide, N-methylpyrrolidone and dimethyl acetamide.

13. The method for preparing olaparib Form H according to claims 10 to 12, **characterized in that** a crystalline or amorphous form of olaparib is stirred in a mixture of water and said organic solvent at a temperature in the range of -20°C to 50°C for 1 to 100 hours.

14. Use of olaparib Form H for the preparation of a pharmaceutical composition with controlled releasing.
